# EUROPEAN PATENT SPECIFICATION

(11) **EP 2 802 648 B1**
(45) Date of publication and mention of the grant of the patent: **28.08.2019**
(21) Application number: 13736077.2
(22) Date of filing: 11.01.2013
(51) Int. Cl.: C12N 5/0735, C12N 5/074, C12N 15/12, C12N 15/861, A61K 48/00, A61P 35/00, C07K 14/82

(54) **CANCER-SPECIFIC SUICIDE GENE FOR CELL-BASED AND GENE THERAPY**
KREBSSPEZIFISCHES SUIZIDGEN FÜR EINE ZELLBASIERTE GENTHERAPIE
GÈNE SUICIDE SPÉCIFIQUE AU CANCER DESTINÉ À UNE THÉRAPIE BASÉE SUR DES CELLULES ET GÉNIQUE

(30) Priority: 13.01.2012 US 201261586366 P
(43) Date of publication of application: 19.11.2014
(73) Proprietor: Sloan Kettering Institute For Cancer Research, New York, NY 10065 (US)
(72) Inventor: ORRICHIO, Elisa, New York, NY 10065 (US); WENDEL, Hans-Guido, New York, NY 10065 (US)
(74) Representative: Pearl Cohen Zedek Latzer Baratz UK LLP
(86) International application number: PCT/US2013/021316
(87) International publication number: WO 2013/106774

(56) References cited:
- US-A1- 2010 303 775
- A. KUMAR ET AL: "c-Myc Is Essential but Not Sufficient for c-Myb-mediated Block of Granulocytic Differentiation", JOURNAL OF BIOLOGICAL CHEMISTRY, vol. 278, no. 13, 13 January 2003 (2003-01-13), pages 11480-11488, XP055159484, ISSN: 0021-9258, DOI: 10.1074/jbc.M300080200
- L SOUCEK ET AL: "Omomyc expression in skin prevents Myc-induced papillomatosis", CELL DEATH AND DIFFERENTIATION, vol. 11, no. 9, 14 May 2004 (2004-05-14), pages 1038-1045, XP055159486, ISSN: 1350-9047, DOI: 10.1038/sj.cdd.4401443
- URI BEN-DAVID ET AL: "The tumorigenicity of human embryonic and induced pluripotent stem cells", NATURE REVIEWS CANCER, vol. 11, no. 4, 10 March 2011 (2011-03-10) , pages 268-277, XP055099905, ISSN: 1474-175X, DOI: 10.1038/nrc3034
- PAUL S. KNOEPFLER: "Deconstructing Stem Cell Tumorigenicity: A Roadmap to Safe Regenerative Medicine", STEM CELLS, vol. 27, no. 5, 1 May 2009 (2009-05-01), pages 1050-1056, XP055070190, ISSN: 1066-5099, DOI: 10.1002/stem.37
- MAURO SAVINO ET AL: "The Action Mechanism of the Myc Inhibitor Termed Omomyc May Give Clues on How to Target Myc for Cancer Therapy", PLOS ONE, vol. 6, no. 7, 21 July 2011 (2011-07-21), page e22284, XP055122386, DOI: 10.1371/journal.pone.0022284
- SOUCEK LAURA ET AL: "Omomyc, a potential Myc dominant negative, enhances Myc-induced apoptosis", CANCER RESEARCH, AMERICAN ASSOCIATION FOR CANCER RESEARCH, US, vol. 62, no. 12, 15 June 2002 (2002-06-15) , pages 3507-3510, XP002705287, ISSN: 0008-5472
- DEBORAH SILVERA ET AL: "Translational control in cancer", NATURE REVIEWS CANCER, vol. 10, no. 4, 1 April 2010 (2010-04-01), pages 254-266, XP055194575, ISSN: 1474-175X, DOI: 10.1038/nrc2824
- N. M. SODIR ET AL: "Endogenous Myc maintains the tumor microenvironment", GENES & DEVELOPMENT, vol. 25, no. 9, 8 April 2011 (2011-04-08), pages 907-916, XP055159495, ISSN: 0890-9369, DOI: 10.1101/gad.2038411
- M. JAIN: "Sustained Loss of a Neoplastic Phenotype by Brief Inactivation of MYC", SCIENCE, vol. 297, no. 5578, 5 July 2002 (2002-07-05), pages 102-104, XP055194601, ISSN: 0036-8075, DOI: 10.1126/science.1071489
- PONZIELLI R ET AL: "Cancer therapeutics: Targeting the dark side of Myc", EUROPEAN JOURNAL OF CANCER, PERGAMON, vol. 41, no. 16, 1 November 2005 (2005-11-01), pages 2485-2501, XP027785617, ISSN: 0959-8049 [retrieved on 2005-11-01]
- TAKUYA FUKAZAWA: "Inhibition of Myc Effectively Targets KRAS Mutation-positive Lung Cancer Expressing High Levels of Myc", ANTICANCER RESEARCH, vol. 30, no. 10, 1 October 2010 (2010-10-01), pages 4193-4200, XP055159488,
- A NIENHUIS ET AL: "Genotoxicity of Retroviral Integration In Hematopoietic Cells", MOLECULAR THERAPY, vol. 13, no. 6, 1 June 2006 (2006-06-01), pages 1031-1049, XP055195647, ISSN: 1525-0016, DOI: 10.1016/j.ymthe.2006.03.001
- ELISA ORICCHIO ET AL: "A Cell Engineering Strategy to Enhance the Safety of Stem Cell Therapies", CELL REPORTS, vol. 8, no. 6, 1 September 2014 (2014-09-01), pages 1677-1685, XP055194594, ISSN: 2211-1247, DOI: 10.1016/j.celrep.2014.08.039
- ANDREW S LEE ET AL: "Tumorigenicity as a clinical hurdle for pluripotent stem cell therapies", NATURE MEDICINE, vol. 19, no. 8, 6 August 2013 (2013-08-06) , pages 998-1004, XP055194597, ISSN: 1078-8956, DOI: 10.1038/nm.3267
- KUMAR, ATUL ET AL.: 'c-Myc is essential but not sufficient for c-Myc- mediated block of granulocytic differentiation' THE JOURNAL OF BIOLOGICAL CHEMISTRY vol. 278, no. 13, 28 March 2003, pages 11480 - 11488, XP055159484
- SOUCEK, LAURA ET AL.: 'Omomyc, a potential myc dominant negative, engances myc-induced apoptosis' CANCER RESEARCH vol. 62, no. 12, 15 June 2002, pages 3507 - 3510, XP002705287
- SOUCEK, L. ET AL.: 'Omomyc expression in skin prevents Myc-induced papillomatosis' CELL DEATH AND DIFFERENTIATION vol. 11, 14 May 2004, pages 1038 - 1045, XP055159486
- SAVINO, MAURO ET AL.: 'The action mechanism of the Myc inhibitor termed Omomyc may give clues on how to target Myc for cancer therapy' PLOS ONE vol. 7, 21 July 2011, pages 1 - 16, XP055122386
- FUKUZAWA, TAKUYA ET AL.: 'Inhibition of Myc effectively targets KRAS mutation-positive lung cancer expressing high levels of Myc' ANTICANCER RESEARCH vol. 30, no. 10, October 2010, pages 4193 - 4200, XP055159488
- SODIR, NICOLE M. ET AL.: 'Endogenous Myc maintains the tumor micro environment' GENES & DEVELOPMENT vol. 25, no. 9, 08 April 2011, pages 907 - 916, XP055159495

## Description

### FIELD OF THE INVENTION

The present invention relates to pluripotent and multipotent stem cells, including embryonic stem cells, induced pluripotent stem cells, adult stem cells and other progenitor cells, that have been modified to contain an inducible cancer-specific suicide gene construct that, upon induction, selectively kills stem- or progenitor-cell-derived cancerous cells without significant effect on healthy tissues or other (noncancerous) cells derived from such cells. This suicide gene construct expresses a dominant negative MYC-interfering protein (D-MIP) that acts as a tumor suppressor in cancer cells without significant deleterious effects on healthy cells and tissues. The suicide gene construct can also be used in gene therapies that produce cancers arising in association with the presence of the gene therapy vector and likewise discriminates between killing of cancerous cells and non-cancerous cells modified with a gene therapy vector.

### BACKGROUND OF THE INVENTION

The versatility of embryonic stem (ES) cells and induced pluripotent stem (iPS) cells to replace and restore any tissue in the body comes in tandem with an increased risk of cancer. An increased cancer risk has also been associated with gene therapy. Hence, reprogrammed tissues, whether derived from ES cells or iPS cells (Takahashi 2006, Wernig 2010, Hanna 2010) or from other multipotent or progenitor cell, as well as from cells treated with gene therapy vectors, present a safety concern (Knoepfler 2009, Shih 2007, Okita 2007, Nakagawa 2010). For example, subcutaneously implanted iPS cells cause teratomas, and iPS chimeric animals develop primitive malignant cancers with high incidence (Takahashi 2006, Knoepfler 2009, Shih 2007, Okita 2007). While benign teratomas may readily be removed by surgery, invasive cancers remain a risk with cell therapies.

Strategies for overcoming stem cell tumorigenicity, including a suicide gene strategy, have been considered (Knoeplfer 2009). However, conventional suicide gene strategies cannot distinguish between benign and malignant tissues and will destroy both benign and malignant tissue when activated (Shuldiner 2003). For example, the approach of introducing a herpes virus thymidine kinase gene (tk) into human ES cells and treating with ganciclovir upon the occurrence of cancer results in killing all tk-expressing cells, cancerous and non-cancerous alike. To overcome this problem, Knoepfler (2009) suggested using a stem cell-specific promoter to control tk expression but recognized the drawbacks of this strategy, namely that the now differentiated host cell may shut off stem-cell promoters, making them inoperable when needed, and further, even if the promoter were operable, the patient would potentially need lifelong ganciclovir treatment (or other suicide-gene inducer as appropriate). Hence, this approach also fails to be cancer-specific.

The connection between pluripotency, differentiation and tumorigenesis is not completely understood. Oncogenic transcription factors, including MYC, KLF4, OCT4 or SOX2, are needed to efficiently reprogram somatic cells (Takahashi 2006, Wernig 2010, Hanna 2010, Shachaf 2004, Jain 2002), and reactivation of MYC may account for most cancers in iPS chimeras (Okita 2007). However, cancers have also occurred when iPS cells were generated without MYC (Takahashi 2006, Wernig 2010, Hanna 2010, Nakagawa 2010, Wernig 2008).

Genetic studies in animal models of cancer have revealed a broad requirement for endogenous MYC activity in most tumors and cancers of various tissue origins (Soucek 1998, Sou cek 2008, Felsher 1999, Shachaf 2004, Jain 2002). Yet, effective drugs targeting MYC are not currently available. Recently, a dominant-negative MYC mutant, Omomyc, has been shown to enhance MYC-induced apoptosis in myoblasts (Soucek 2002) and to act as a suppressor of MYC-induced oncogenesis in animal models of papillomatosis (Soucek 2004), lung cancer (Fukazawa 2011) and pancreatic cancer (Sodir 2011). Hence, Omomyc has been found to be tumor suppressive in cancer.

Omomyc forms transcriptionally inactive heterodimers with endogenous MYC proteins (c-MYC, N-MYC and L-MYC) and blocks MYC-MAX interaction (Soucek 1998, Soucek 2002, Soucek, 2004, Soucek 2008). Savino (2011), in a study on the mechanism of Omomyc action, suggested that Omomyc might play a role in discovering small molecule anticancer therapies, recognizing Omomyc only "as a tool that models the efficacy of future strategies that interfere with MYC function in tumorigenesis." Another group (Van Eyss 2011) postulated that Omomyc's value was only as a research tool for discovery of therapeutics targeting MYC. Hence neither recognized the potential direct therapeutic efficacy of the Omomyc protein.

However, the present inventors have found that Omomyc can serve directly as a therapeutic agent to provide a tumor-selective (or cancer-specific) approach to eradicate cancerous cells arising from reprogrammed tissues without lasting disruption of differentiated or normal regenerative tissues in transgenic and iPS chimeric animals. Hence, the present invention provides an avenue to enhance the safety of reprogrammed tissues in regenerative medicine using a cancer-specific suicide gene construct.

### SUMMARY OF THE INVENTION

The present invention relates to the use of a dominant-negative, MYC interfering protein (D-MIP, defined below) in a suicide construct for treating cancer and/or reducing tumorigenesis in cell-based therapy and gene therapy.

Accordingly, the present invention provides an agent for use in reducing tumorigenesis or tumor growth in a MYC-dependent cancer occurring during the course of gene therapy in a subject, wherein the subject has been administered cells that have been transduced *ex vivo* with a gene therapy vector comprising a therapeutic gene operably linked to a first control element, and a dominant-negative MYC-interfering protein (D-MIP) gene operably linked to an inducible control element, wherein the MYC-dependent cancer is derived from the cells that have been administered to the subject for gene therapy, wherein the agent is an inducer of the inducible control element, and wherein, upon detection of cancer in the subject, the inducer is administered to the subject for a time and in an amount to activate D-MIP in the subject, thereby reducing tumorigenesis or tumor growth of the MYC-dependent cancer.

In an embodiment, the inducible control element is a mutant hormone binding domain of the estrogen receptor and the inducer is tamoxifen.

In an embodiment, the inducible control element is a cumate switch inducible system and the inducer is cumate; the inducible control element is a glucocorticoid-responsive promoter and the inducer is a glucocorticoid; the inducible control element is a tetracycline response element and the inducer is a tetracycline such as doxycycline; the inducible control element is a hormone receptor ligand binding domain and the inducer is a hormone receptor ligand; or wherein the inducible control element is an HSV viral protein VP16 activator domain and the inducer is an HSV VP16 activator protein.

In an embodiment, the gene therapy vector is a lentiviral vector for treating severe combined immunodeficiency disease (SCID) or for treating a hemoglobinopathy such as thalassemia or sickle cell disease.

In an embodiment, the MYC-dependent cancer is bladder cancer, breast cancer, colon cancer, gastric cancer, hepatocellular carcinoma, leukemia, lymphoma, melanoma, myeloma such as multiple myeloma, neuroblastoma, ovarian cancer, prostate cancer, rhabdomyosarcoma, small cell lung cancer, subungual melanoma, or uveal melanoma, and wherein the Myc-dependent cancers is metastatic or non-metastatic.

In an embodiment, the gene therapy vector is a lentiviral vector, an adenoviral vector or an adeno-associated viral vector.

In an embodiment, the D-MIP is Omomyc or in another embodiment the D-MIP and second control element together form Omomyc^{ER}.

In an embodiment, the administered cells are hematopoietic stem cells, hematopoietic progenitor cells, iPS cells or ES cells.

In an embodiment, the cells are autologous cells from said subject.

In an embodiment, the subject is human.

The administered cells may be isolated pluripotent stem cells, multipotent stem cells or progenitor cells which comprise an inducible D-MIP construct, typically but not necessarily, incorporated into the genome of those cells. The stem cells include mammalian iPS cells and ES cells, as well as adult stem cells (including hematopoietic, pulmonary, hepatic, exo-endocrine, pancreatic and thymic stem cells), tissue-specific stem cells, organ-specific stem cells, fetal stem cells and cord blood stem cells. Progenitor cells include hematopoietic progenitor cells, skin progenitor cells, bone progenitor cells, and neural progenitor cells, as well as hepatic, exo-endocrine, pancreatic, pulmonary, thymic and other tissue and organ specific progenitor cells.

Also disclosed herein is a method of reducing tumorigenesis or tumor growth in a MYC-dependent cancer in a subject by introducing therapeutically-useful stem cells, reprogrammed cells or tissue derived from such stem or reprogrammed cells into a subject, where such cells or tissue comprises an inducible D-MIP construct, and if cancer is detected or suspected, treating the subject with an inducer of the D-MIP construct for a time and in an amount to express or activate D-MIP in the subject and thereby reduce tumorigenesis or tumor growth. The stem cells, reprogrammed cells or tissues are prepared from iPS cells, ES cells, hematopoietic progenitor cells, skin progenitor cells, bone progenitor cells or neural progenitor cells. The method is preferably used with mammals, including both non-human mammals (e.g., primates) and humans. In certain embodiments the D-MIP is Omomyc or Omomyc^{ER}.

The gene therapy vector comprises a therapeutic gene operably linked to a first control element and a D-MIP construct operably linked to a second, inducible control element. It is also possible for the therapeutic gene and the D-MIP to be under control of the same promoter in instances where the D-MIP activity is inducible in trans, such as when using a hormone binding domain fused to the D-MIP (as in Omomyc^{ER}). The D-MIP may be Omomyc or Omomyc^{ER}.

It is disclosed that the gene therapy vectors can be used in methods of reducing tumorigenesis or tumor growth in a MYC-dependent cancers in a subject by administering a gene therapy vector to the subject; and if cancer is detected or suspected, treating the subject with an inducer of the D-MIP construct for a time and in an amount to express or activate D-MIP in the subject and thereby reduce tumorigenesis or tumor growth. The gene therapy vectors can also be used in similar methods in which cells are transduced *ex vivo* with a gene therapy vector, the transduced cells are introduced into the subject and treatment proceeds as when delivering the gene therapy vector directly to the subject. In a preferred embodiment, the gene therapy vector is used to transduce autologous cells of the subject. While *ex vivo* applications typically are done with hematopoietic stem cells or hematopoietic progenitor cells, iPS cells or ES cells can also be reprogrammed into various hematopoietic cells, transduced with a gene therapy vector and used in any of these disclosed methods. The D-MIP may be Omomyc or Omomyc^{ER}.

Also disclosed herein are methods of reducing tumorigenic potential of iPS or ES cells. For example, once can transduce iPS or ES cells with an inducible D-MIP construct, induce D-MIP activity before or after reprogramming and select for surviving cells. The surviving cells have lowered tumorigenic potential. Alternatively, for use in a subject, the cells can be reprogrammed after transduction, D-MIP activity induced and the surviving (differentiated) cells implanted into a subject in need of the therapy for which the cells were reprogrammed. In another embodiment, the reprogrammed cells can be implanted into a subject for therapeutic treatment and D-MIP activity induced after detection of cancer arising from such cells. In preferred embodiments, these methods are used with autologous iPS cells and D -MIP is Omomyc or Omomyc^{ER}.

### BRIEF DESCRIPTION OF THE DRAWINGS

Fig. 1 schematically illustrates generation of iPS cells for inducible MYC regulation. MEF, mouse embryo fibroblasts; Vector, lentiviral control vector, Omomyc^{ER}, vector encoding Omomyc in frame to a mutant hormone binding domain of the estrogen receptor (Littlewood 1995) to confer tamoxifen-inducible control of Omomyc activity; iPS^{OMO}, iPS cells containing Omomyc^{ER}.
Fig. 2 shows a fluorescent-activated cell sorter (FACS) analysis of MEFs transduced with KLF4, OCT4, SOX2 and MYC, each transcriptionally linked to a different fluorescent marker.
Fig.3a schematically illustrates a lentiviral construct encoding a tamoxifen-inducible Omomyc^{ER} fusion protein and a citrine reporter. Fig. 3b shows the plasmid used to prepare the lentiviral vector.
Fig. 4, top panels, shows FACS analysis of control iPS cells and citrine-expressing iPS^{OMO} cells. SSC, side scatter. Bottom panels show FACS analysis of control and Omomyc^{ER} / citrine expressing human-iPS cells clone 5.10-omo10.
Fig.5 depicts three bar graphs with quantitative immunohistochemical analysis of teratoma and embryonal carcinomas (EmbryonalCA) derived from control iPS cells (control) or Omomyc^{ER}-expressing iPS cells (Omo) for Ki67 staining (Fig. 5a), for TUNEL staining (Fig. 5b) and for SALL4 staining (Fig. 5c).
Fig. 6 presents bar graphs with the results of quantitative RT-PCR measuring expression of exogenous (human) (b) and endogenous (murine) (c) cMYC expression in mouse embryo fibroblasts (MEF) and embryonal carcinomas.
Fig. 7a photographically illustrates representative tumors in NOD/SCID mice injected with p53-/- iPS cells expressing Omomyc^{ER} (iPS^{OMO}) or with control p53-/- iPS cells (iPS^{Control}), left and right panels respectively. Tumors were harvested following 3 weeks of tamoxifen treatment. Fig 7b graphically depicts tumor weight (g) after tamoxifen treatment.
Fig. 8a photographically illustrates representative tumors in NOD/SCID mice injected with p53+/+ iPS cells expressing Omomyc^{ER} (iPS^{OMO}) or with control p53+/+ iPS cells (iPs^{Control}), left and right panels respectively. Tumors were harvested following 3 weeks of tamoxifen treatment. Fig 8b graphically depicts tumor weight (g) after tamoxifen treatment.
Fig. 9 shows photographs of a chimeric mouse before (top) and after (bottom) a 3 week course of tamoxifen (indicated as TAM in the figure) to activate Omomyc expression in iPS cell-derived tissues. The black skin patch originates from the iPS donor cells.
Fig. 10 is a graph indicating the percent survival of mice receiving control cells (T-ALL/control) or Omomyc^{ER} cells T-ALL/OMOMYC).
Fig. 11 shows a FACS plot of GFP fluorescence for cells obtained from the bone marrow of T-ALL/Omomyc and T-ALL/control mice on day 3 of tamoxifen treatment.
Fig. 12 is a bar graph depicting hMYC relative expression level in primitive neuroectodermal tumors (PNET) and healthy differentiated neurons.
Fig. 13 schematically illustrates a cell engineering approach to exploit differential MYC dependence in cancerous and healthy tissue.

### DETAILED DESCRIPTION OF THE INVENTION

### Overview and Definitions

The present invention provides agents that selectively kill cancer cells arising from reprogrammed tissues or arising during the course of gene therapy. In accordance with the present invention, conditional MYC inactivation discriminates between cancerous and differentiated (or otherwise non-cancerous) tissues and provides a suicide mechanism to target cancer cells arising from reprogrammed tissues or arising during gene therapy without significant adverse affects on the remaining or healthy tissue. Since most tumors are MYC-dependent, this has wide applicability in the treatment of MYC-dependent cancers. The suicide strategy involves introducing an inducible dominant-negative Myc-Interfering Protein (D-MIP) genetic construct into a stem or progenitor cell or providing an inducible D-MIP genetic construct as part of a therapeutic gene vector. In either case, the D-MIP is activated when needed to treat a MYC-dependent cancer. Thus, introducing a D-MIP construct into stem cells (or into a gene therapy vector) under inducible control provides a tumor-selective, suicide mechanism for use in cell and gene therapies.

The present invention thus provides an agent for use in reducing tumorigenesis or tumor growth in a MYC-dependent cancer occurring during the course of gene therapy in a subject, wherein the subject has been administered cells that have been transduced *ex vivo* with a gene therapy vector comprising a therapeutic gene operably linked to a first control element, and a dominant-negative MYC-interfering protein (D-MIP) gene operably linked to an inducible control element, wherein the MYC-dependent cancer is derived from the cells that have been administered to the subject for gene therapy, wherein the agent is an inducer of the inducible control element, and wherein, upon detection of cancer in the subject, the inducer is administered to the subject for a time and in an amount to activate D-MIP in the subject, thereby reducing tumorigenesis or tumor growth of the MYC-dependent cancer.

As used herein, a "dominant-negative MYC-interfering protein," or "D-MIP," is a genetically-encoded protein or peptide that prevents Myc-Max binding to its DNA targets and inhibits MYC transactivation activity while retaining MYC transrepression activity. The D-MIP also has tumor suppressive activity. The D-MIP is incorporated into a genetic construct in a manner to provide inducible control of the D-MIP expression or activity. This can be accomplished in ways known to those of skill in the art, for example using an inducible promoter to control expression or having the protein be responsive to an inducer that leads to activation of D-MIP activity. In any case, with the D-MIP under inducible control, it can be expressed or activated under specific conditions or at specific times (as determined or predetermined by the practitioner), and is preferably activated when cancerous cells occur during the course of a cell-based therapy or a gene therapy with cells or a vector carrying the inducible construct. The D-MIP suicide construct is thus introduced into a cell or incorporated into a gene therapy vector for use in accordance with the present invention. Omomyc and Omomyc^{ER} are examples of D-MIPs. Further examples of D-MIPs also include transcription factor alleles that act to modify gene expression, dominant negative inhibitors of signaling pathways (e.g., inhibitory alleles of extracellular and intracellular receptors), and dominant inhibitors of protein translation like eIF4E-BP and its constitutively active alleles).

"Omomyc" means the protein described in Soucek (1998) or any equivalently functioning variant thereof such as might be obtained by allelic variation in the MYC coding sequence, so long as the Omomyc mutations (and hence Omomyc activity) are retained. Omomyc is human MYC with four mutations introduced into its leucine zipper region; the mutations are E57I, E64I, R70Q and R71N (which means that the glutamate at position 57 has been changed to isoleucine, the glutamate at position 64 has also been changed to isoleucine, and the arginines at positions 70 and 71 have been changed to glutamine and asparagine, respectively). Omomyc forms homodimers as well as heterodimers with c-MYC, N-MYC, MAX and MIZ-1. Omomyc's ability to bind MYC and MAX leads to sequestering of MYC and to inhibition of MYC's transcriptional activator function in the Max network, also referred to as the Myc interactome (Meyer 2008; Savino 2011). Omomyc is also tumor suppressive. Hence, Omomyc acts as a dominant negative MYC mutant (*i*.*e*., a D-MIP). Omomyc can be conditionally activated, for example, by placing its expression under control of an inducible promoter.

Omomyc^{ER} is a fusion protein of Omomyc with the mutant hormone binding domain of the estrogen receptor (Littlewood 1995). In the absence of tamoxifen, Omomyc^{ER} is present in the cell in inactive complexes (having been expressed by a constitutive promoter). In the presence of tamoxifen, the inactive Omomyc^{ER} complexes dissociate, Omomyc becomes active and exerts D-MIP activity as described herein.

The D-MIPs may or may not be species-specific. MYC is highly conserved across species and cross-species activity may be possible. Those of skill in the art can readily determine if cross-species activity is possible. For example, Omomyc, based on the human MYC sequence, functions in mice and murine cells, as well as in human cells. Undesired immunogenicity problems can be avoided by using species-specific proteins.

The "inducible D-MIP construct" comprises one or more "inducible" control elements that can be activated by a specific signal or agent upon occurrence of a specific event to thereby express the D-MIP. These control elements can be cis-acting or trans-acting. For example, an inducible promoter is a cis-acting genetic control element that, in response to a specific induction signal, activates transcription of the D-MIP coding sequence to which it is operably linked. Those of skill in the art can readily make an inducible D-MIP construct using recombinant DNA techniques and known inducible control elements. In the case of Omomyc, its coding sequence is described in Soucek (1998). Selection of an appropriate combination of control elements to provide an inducible D-MIP construct is within the ken of the art.

Genetic control elements include, but are not limited to, promoters, enhancers, derepressor elements and the like, and may be used singly or in combination to provide inducible expression. Examples of inducible promoters and control elements include, but are not limited to, the cumate switch inducible system (SparQ™ Cumate Switch, System Biosciences), glucocorticoid-responsive promoters, as well as the tetracycline response elements (TREs) which can be induced with doxycycline. Such promoters can include both strong promoters and tissue-specific promoters. For example, tissue-specific promoters can limit activation to certain cell lineages (*e.g*., hematopoietic - vav promoter; hepatic - albumin promoter) or to certain cell stages (*e.g*., cells expressing CD34 or other stage-specific markers). Sequences of inducible or conditionally active promoters or other control elements are known to those of skill in the art as are techniques for preparing and purifying vectors with the D-MIP coding sequence operably linked to a genetic control element.

Inducible activity can also be achieved, for example, using hormone inducible fusion proteins in which the hormone receptor ligand binding domain is fused to the D-MIP. An example of such a domain is the mutant estrogen receptor domain (ER) which responds to tamoxifen (Littlewood 1995). Omomyc^{ER} has this mutant ER domain fused at its C terminus (Soucek 2002). With hormone-inducible fusions, the gene construct can be under the control of a strong constitutive promoter. The expressed fusion protein is sequestered in an inactive state until addition of tamoxifen, whereupon the fusion protein is released, allowing the D-MIP domain to exert its activity. Other activatable domains that can be used as fusion proteins with Omomyc or other D-MIPs, include, but are not limited to, the HSV viral protein VP16 activator domain, engineered nucleotide sequences that target transcriptional regulators to specific sites (e.g., TALENs), and site-specific molecules to target genomic DNA (e.g., engineered and natural zinc-finger proteins).

### Cells

The administered cells may be isolated mammalian pluripotent stem cell, multipotent stem cell or progenitor cell that comprises an inducible D-MIP suicide construct. In preferred embodiments the cells are iPS cells or ES cells, and the D-MIP construct is an inducible Omomyc gene construct.

The stem and progenitor cells (collectively referred to herein as "stem cells") include adult stem cells or tissue-specific stem cells, fetal stem cells, cord blood stem cells, embryonic stem (ES) cells, and induced pluripotent stem (iPS) cells. Examples of tissue-specific stem cells include, but are not limited to, hemopoietic stem cells, hemopoietic progenitor cells, peripheral blood stem cells and mesenchymal stem cells. Preferred pluripotent stem cells are iPS cells, and more preferably, patient-specific iPS cells, *i*.*e*., iPS cells derived from autologous somatic cells. The pluripotent and multipotent cells can generally be identified by cell-surface markers using methods known to those of skill in the art. In other preferred embodiments, the stem cells are ES cells, multipotent hematopoietic stem cells or hematopoietic progenitor cells.

Any of the stem or progenitor cells can be from a mammal, which includes non-human mammals and humans. Non-human mammals include, but are not limited to, primates, mice, cows and cattle, sheep, goats, dogs and rats.

Methods for transducing stem and progenitor cells with inducible D-MIP constructs are known in the art, and representative methods therefor are provided in the Examples.

### Gene Therapy Vectors

The gene therapy vector comprises an inducible D-MIP gene construct as described above. These vectors contain a therapeutic gene and an inducible D-MIP gene construct as separate and generally independently-controlled genetic units in the vector, especially if an inducible promoter is used to control transcription of the D-MIP. However, for example, if the D-MIP is a hormone inducible fusion protein, the therapeutic gene and the D-MIP can be coordinately expressed if desired. Gene therapy vectors are known in the art and include, but are not limited to, lentiviral vectors, adenoviral vectors, adeno-associated viral vectors, plasmids and the like. Construction of a gene therapy vector can be done by methods known in the art. In a preferred embodiment, the inducible D-MIP construct is an inducible Omomyc construct.

As an example of a gene therapy vector, an inducible D-MIP construct can be incorporated into a gene therapy vector, for example, the D-MIP construct can be included in a lentiviral vector. Since leukemia has been associated with certain gene therapies, incorporation of a vector with a D-MIP suicide construct allows killing of leukemic cells upon treatment with the appropriate inducer. In this case, the D-MIP- containing therapeutic vector can be transduced into cells *ex vivo* and the cells delivered to the patient. Likewise, a therapeutic vector can be delivered directly to the patient.

### Methods

The invention relates to reducing tumorigenesis or tumor growth in MYC-dependent cancers arising in the course of treatment with reprogrammed cells or tissue in a mammalian subject, including a human patient. For example, inducible D-MIP constructs can be introduced into iPS, ES cells or the other stem cells at any convenient stage during their preparation for reprogramming or therapeutic use. Once the cells or tissue (if generated) are established, the therapeutically-useful reprogrammed cells or tissue are used for treatment of the targeted disease or condition. The subject or patient is monitored and if cancer is detected or suspected, then the subject or patient is treated with an appropriate inducer to activate the D-MIP. For example, when using Omomyc^{ER}, the patient is treated with tamoxifen using a dosage which can be determined by those of skill in the art. For example, well-tolerated tamoxifen doses for breast cancer and reduction of risk of breast cancer are 20-40 mg/day, which is approximately 0.3-0.6 mg/kg/day for a 70 kg person. Hence, tamoxifen can be used as an inducer in amounts ranging from 0.1 to 1 mg per kg body weight per day. The appropriate dosages for other inducers can be also be so determined by analogy with the tolerated doses when those inducers are used for other purposes (*e.g*., the appropriate doxycycline dose can be analogized from its use as an antibiotic).

The invention also relates to reducing tumorigenesis or tumor growth in MYC-dependent cancers arising in association with, or during the course of, gene therapy in a subject or patient. A therapeutic vector with an inducible D-MIP construct is administered to the subject or patient, and the therapeutic efficacy with respect to the targeted disease or condition is monitored as well as the general health of the subject or patient. If cancer is detected or suspected, then the subject or patient is treated with an inducer to activate D-MIP expression or activity. Dosages of inducer in gene therapy applications are similar to those used for cell-based therapies.

The invention also relates to reducing tumorigenic potential of iPS or ES cells by inserting (generally by transduction) an inducible D-MIP construct into those cells and inducing D-MIP activity. Such cells can be used for therapeutic purposes before or after inducing D-MIP activity. For example, such cells can be differentiated to a particular cell type for a target therapy and implanted into a patient in need of that therapy. Thereafter, the suicide construct can be activated if cancer is detected.

The stem cells can be used for any therapeutic condition or disease amenable to treatment with reprogrammed tissues or cells, or to treatment with stem cells, progenitor cells and the like, including iPS cells and ES cells. For example, suicide D-MIP expression can be engineered into iPS-derived neurons to treat a neurological disorder. If a tumor arises, then expression or activation of the D-MIP is induced to cause tumor shrinkage or remission.

The diseases and conditions amenable to stem cell therapy include, but are not limited to, stroke, brain injury, Alzheimer's disease, Parkinson's disease, spinal cord injury, burns, heart disease, Crohn's disease, diabetes, muscular dystrophies, ALS (Lou Gehrig's disease), osteoarthritis, rheumatoid arthritis, and many more. The present invention allows one to prepare stem cells that can be selectively killed if these cells are reprogrammed and then generate cancerous cells after transplantation. To accomplish this, one identifies or prepares a relevant stem cell population, transduces those cells with an inducible D-MIP construct, treats the cells to differentiate or otherwise prepares those cell for transplantation (e.g., prepares a tissue) and then transplants the cells (or prepared tissue) into the patient. The patient is then monitored for the occurrence of cancer or for any other oncogenic signal. Once a cancer or oncogenic signal is detected, the patient is treated with an agent to induce expression of D-MIP for a time and in an amount to kill cancer cells arising from the transplanted cells (or tissue as the case may be). Alternatively, the stem cells can be prepared for their therapeutic purpose, e.g., allowed to differentiate, and those differentiated cells can be transduced with an inducible D-MIP construct before the cells are implanted or transplanted.

As used herein, cancer means MYC-dependent cancer. MYC-dependent cancers include, but are not limited to, bladder cancer, breast cancer, colon cancer, gastric cnacer, hepatocellular carcinoma, leukemia, lymphoma, melanoma, myeloma (including multiple myeloma), neuroblastoma, ovarian cancer, prostate cancer, rhabdomyosarcoma, small cell lung cancer, subungual melanoma, uveal melanoma and the like. Myc-dependent cancers can be metastatic or non-metastatic.

The presence of an inducible D-MIP construct within reprogrammed tissues or in gene therapy vectors does not preclude the use of conventional and known cancer treatments if cancer occurs in conjunction with use of those reprogrammed tissues or gene therapy vectors.

The foregoing is considered as illustrative only of the principles of the invention, and numerous modifications and changes will readily occur to those skilled in the art.

### EXAMPLE 1

### Generation and characterization of p53+/+ and p53-/- iPS Cells

### A. Generation of iPS cells

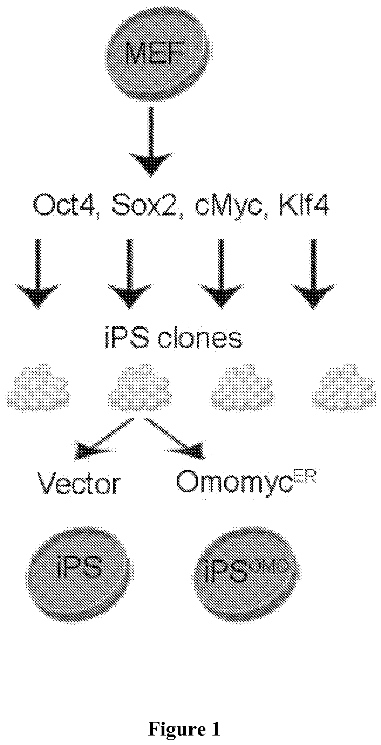

MEFs from p53 +/+ and p53 -/- animals were reprogrammed into iPS cells using a standard protocol (Fig. 1) (Papapetrou 2009). Briefly, 10⁵ MEFs were plated into 6-well plates and transduced 24 h later with four lentiviral vectors (pLM-OCT4, pLM-Sox2, pLM-MYC and pLM-Klf4) in the presence of 4 µg/mL polybrene. Media was changed 24 h later and replaced every day thereafter with mES cell media (Invitrogen) supplemented with 1000 U/mL LIF and 0.5 mM valproic acid (VPA; Sigma).

Single colonies with ES cell morphology were picked 15-25 days after transduction, mechanically dissociated and plated on mitomycin C-treated MEFs (Global Stem) in KnockOut™ DMEM with high glucose (GIBCO), supplemented with 15% Fetal Bovine Serum (Hyclone), 0.1 mM β-mercaptoethanol, 4mM L-glutamine, 1x non-essential amino acids and 1000 U/ml LIF. Media was changed every day. Cells were thereafter passaged with trypsin and expanded to establish iPS cell lines.

### B. iPS cell characterization

For immunofluorescence analysis, iPS cells were grown on glass coverslips and fixed with 4% paraformaldehyde for 10 min at room temperature. Fixed cells were incubated in PBS containing 20% goat serum for 30 min at 37°C in a humidified chamber and then overnight at 4°C with anti-Nanog antibodies (eBioscience) diluted 1:1000 and with anti-SSEA1 antibodies (eBioscience) in PBS containing 5% goat serum. After five washes in PBS with 0.05% Tween 20, nuclei were counterstained with 0.1 mg/ml 4',6'-diamino-2-phenylindole (DAPI) in 2X SSC for 3 min at room temperature. Samples were then washed in PBS and mounted on glass slides in Vectashield® mounting media (Vector Laboratories). Cell preparations were examined under a Leica fluorescence microscope equipped with a cooled CCD-camera (Coolsnap, Photometries).

Flow cytometry analysis and karyotyping assay were performed as described (Papapetrou 2011).

To assess expression of pluripotency genes, total RNA was isolated from cells using Trizol (Invitrogen). Reverse transcription was performed with Superscript III (Invitrogen), and qPCR was performed with a TaqMan® probe-based gene expression assay (Applied Biosystems) using the indicated probes for the genes: Sox2 (Mm03053810), Oct4 (Mm03053917), MYC (Mm00487804), Klf4 (Mm00516104). Reactions were carried out in duplicate in an ABI PRISM 7500 Sequence Detection System (Applied Biosystems). Expression was calculated by relative quantification using the ΔΔCt method

### C. Results

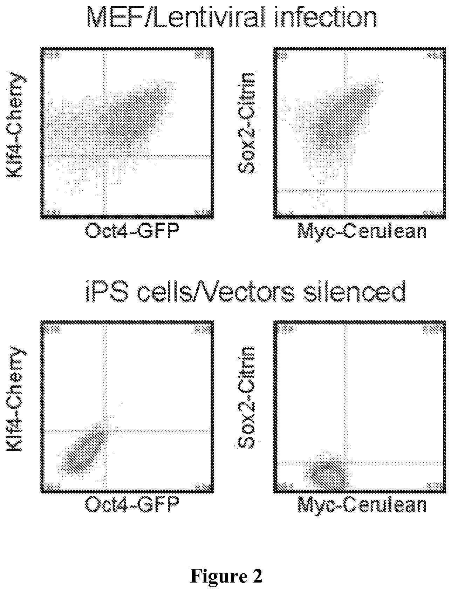

iPS cell colonies developed faster and more efficiently from p53-/- MEFs relative to p53+/+ MEFs cells as previously observed (Hong 2009, Kawamura 2009, Marion 2009). Immunofluorescence showed expression of endogenous Nanog and stage-specific embryonic antigen-1 (SSEA1) in both p53+/+ and p53-/- iPS clones. Expression of Klf4, Sox2, c-MYC, and Oct4 was at or below the levels seen in ES cells. The FACS analysis for iPS cells expressing Klf4, Sox2, c-MYC, and Oct4, each transcriptionally tethered to a different fluorescent reporter, is shown in Fig. 2a (top panels). FACS showed silencing of the exogenous *OCT4, MYC, KLF4*, and *SOX2* linked reporters Fig 2 (bottom panels), and RT-PCR confirmed expression of their endogenous counterparts at or below the level seen in ES cells.

### EXAMPLE 2

### Transduction of p53+/+ iPS cells and p53-/- iPS cells with Omomyc

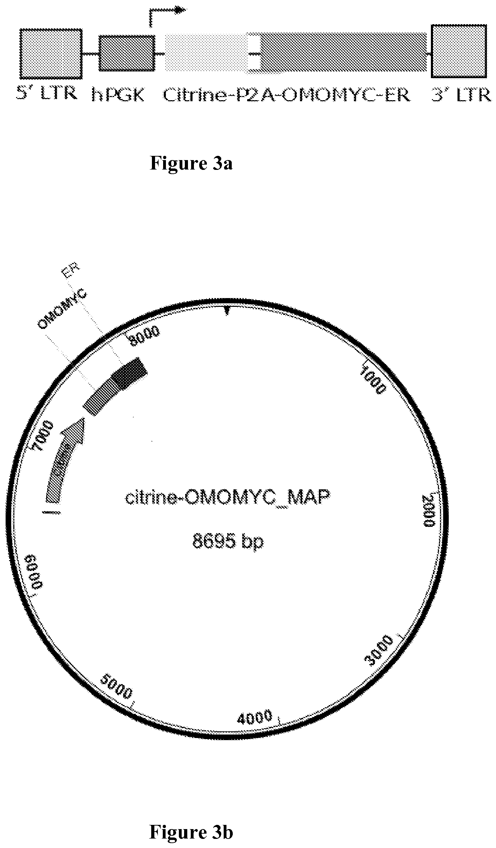

Three clones each of p53+/+ and p53-/- iPS cells with normal karyotype were transduced with a lentivirus encoding a tamoxifen-inducible Omomyc^{ER} fusion protein and a citrine reporter using the viral supernatant from pML-OMOMYC-ER-Citrine. The lentiviral vector was prepared from the plasmid construct shown schematically in Fig. 3a and 3b.

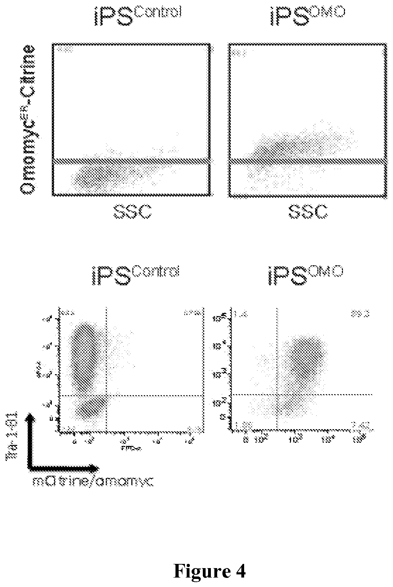

Fig. 4 shows a FACS analysis of control iPS cells (left) and Omomyc^{ER}-citrine iPS cells. The p53+/+ and p53-/- iPS cells containing inducible Omomyc are amenable to temporal control of MYC activity.

### EXAMPLE 3

### In Vivo Tumor Formation from p53+/+ iPS cells and p53-/- iPS cells

### A. Tumor preparation and analysis

To form tumors, 10⁶ undifferentiated iPS cells were injected subcutaneously (s.c.) into NOD-SCID *IL2Rg*^{-null} mice (Jackson Laboratory). Four to six weeks later, tumors were surgically dissected and fixed in 4% formaldehyde.
Tumors were evaluated by an expert in germ cell tumor pathology. Histological diagnosis was based on hematoxylin and eosin staining and immunohistochemistry as generally described in Example 1 using antibodies against Sal4 (Abcam), Oct4 (Ventana) and CD30 (Dako). In treatment studies, tumors were collected one week after the last treatment, weighed, fixed and stained as above.

### B. Results

Subcutaneous injection of p53+/+ iPS cells caused tumors (> 1cm³) within 60 days whereas injection of the same number of p53-/- iPS cells formed tumors in 30 days (n = 6 for each group; p < 0.01). Both p53+/+ derived and p53-/- derived tumors showed equal c-MYC expression by RT-PCR.

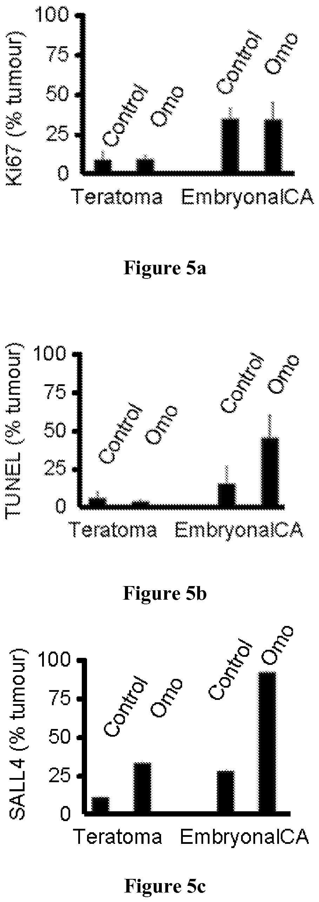

Tumors arising from p53+/+ iPS cells were immature teratomas and contained differentiated tissues representing all three germinal layers. These tumors had a low growth fraction as indicated by Ki67 expression (mean±SD: 8.7% ± 5%) and contained few apoptotic cells as determined by TUNEL assay (mean±SD: 5.6 % ± 3%) (see Figs. 5a and 5b). Few apoptotic cells were observed by cleaved caspase 3 and there was no evidence of MYC re-activation. Immunohistochemistry was negative for markers of pluripotent cells (CD30 and OCT4), and revealed small pockets of cells that stained positive for SALL4, a marker of yolk sac-endodermal sinus differentiation (mean±SD: 11.8% ± 7%) (Fig. 5c). Based on these observations, these tumors were best described as immature teratomas.

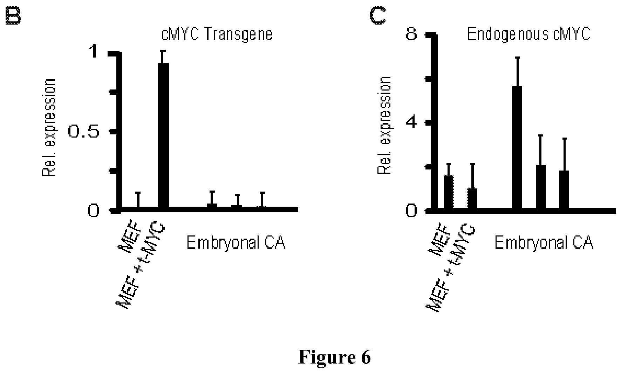

Tumors arising from p53-/- iPS cells were markedly different and morphologically resembled embryonal carcinomas (EC). They were composed of primitive cells and showed areas of differentiation. Consistent with their rapid onset, these tumors had a larger growth fraction by Ki67 staining (mean±SD: 34.6% ± 6%) and somewhat variable apoptosis by TUNEL assay (mean±SD: 14.2% ± 10%) (Fig. 4a and 4b). Oct4 and CD30 stains were negative, and 28% (± 11%) of tumor tissue stained for SALL4, indicating areas of yolk sac differentiation within these morphologically primitive and fast growing cancers (Fig. 5c). The (re-)expression of the exogenous human *MYC* transgene was not detected and the endogenous *MYC* mRNA was elevated by up to 5-fold compared with embryonal fibroblast (Fig. 6). Hence, *p53*-deficient iPS cells give rise to primitive embryonal carcinomas that do not re-activate the exogenous *MYC* construct.

### EXAMPLE 4

### Omomyc Induction Causes Tumor Regression

### A. Tumor Preparation and Induction

About 10⁶ iPS cells expressing Omomyc^{ER} or 10⁶ control cells were injected s.c. into NOD/MrkBomTac-*Prkdc^{scid}* mice (Taconic). When the tumors were well-palpable (∼1 cm³), the mice were treated intraperitoneally with 100 µl of tamoxifen (10 mg/ml in peanut oil) every other day for 2 weeks. Tumors were collected one week after the last treatment, weighed and fixed in 4% formaldehyde for subsequent stains including hematoxylin/eosin and immunohistochemistry as described in Example 3.

### B. Results

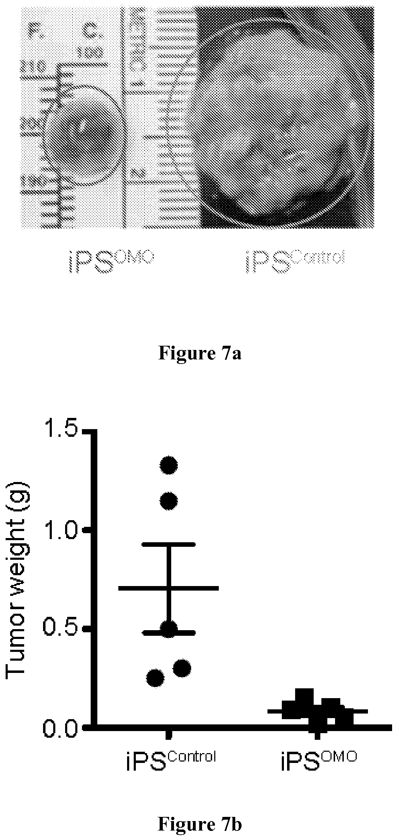

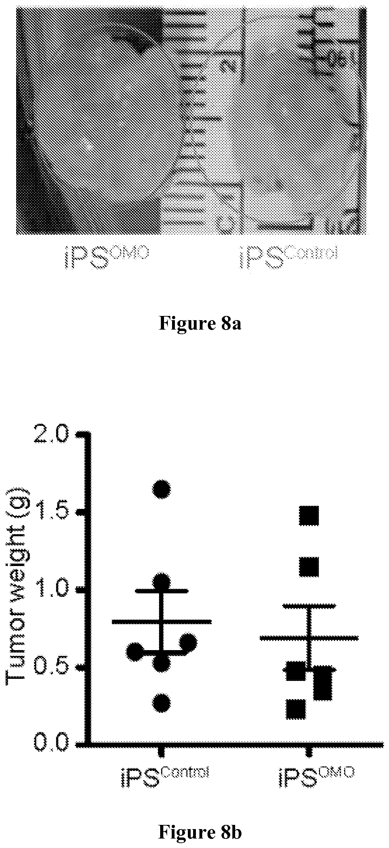

The effect of MYC inhibition on iPS-derived tumors was assessed by transplanting p53-/- iPS cells engineered to express Omomyc^{ER} or p53-/- iPS control cells into the left and right flanks of NOD/SCID mice, respectively (each group, n=5). Tamoxifen treatment produced dramatic tumor regression in tumors expressing Omomyc^{ER}, whereas control tumors continued to grow (p<0.005) as shown in Fig. 7a and 7b). By contrast, in p53+/+ iPS-derived teratomas, MYC blockade produced little effect compared to controls (Figs. 8a and 8b). These differentiated teratomas continued to grow under tamoxifen treatment and showed no significant difference in tumor size or weight one week after the last tamoxifen application (p = 0.7, n=6 for each group).

Tumor microscopy confirmed these observations. For example, the aggressive p53-/- derived tumors had vast areas of apoptosis (TUNEL assay, mean±SD: control 14.2% ± 10% and Omomyc^{ER} 41.2% ± 13%; p< 0.05; Ki67: control 24.2% ± 10% and Omomyc^{ER} 34.6% ± 6%). Taxomifen treatment of Omomyc^{ER}-containing embryonal carcinomas also resulted in a clear shift of the cytological composition, such that the primitive and undifferentiated components had largely disappeared, and the remnants consisted of differentiated, cartilaginous and SALL4-positive tissues (mean±SD: control 28.7% ± 14%, and Omomyc^{ER} 92.3% ± 19%; p < 0.05).

Microscopy revealed few changes in the p53+/+ teratomas. The Ki67-positive growth fraction was 8.7 ± 5%, for control and 9.1 % ± 2.5% for Omomyc^{ER} mice (p > 0.05). The apoptotic indices were unchanged (control 5.6% ± 3.6%, treated 3.6% ± 2%; p > 0.05). Some enrichment of SALL4 positive cells with yolk sac differentiation occurred (control 11.8% ± 7.5%, and Omomyc^{ER} 33.8% ± 15%; p <0.05). Hence, MYC inactivation via Omomyc induction provides a therapeutic reagent against undifferentiated, aggressive cancers arising from reprogrammed cells.

### EXAMPLE 5

### Effect of Omomyc Expression in Healthy iPS-derived tissues

### A. Generation of Chimeric mice with iPS Cells.

Frozen iPS^{OMO} cells from C57B1/6J strain (black coats) were thawed in 6-well plates containing irradiated MEF feeders. When cells reached confluency, they were trypsinized and expanded by passaging into larger culture vessels at a ratio of 1:4 to 1:6. When sufficient cells were available, a small portion was subjected to karyotyping, and the remainder were frozen for future use. In addition, some of the cells were analyzed for alkaline phosphatase (AP) expression. All cells were cultured in medium containing KnockOut™ DMEM with high glucose (GIBCO), supplemented with 15% Fetal Bovine Serum (Gemini), 0.1mM β-mercaptoethanol, 4 mM L-glutamine, 1x non-essential amino acids and 1000U/ml of LIF. During the week of injection, 2 x 10⁶ frozen iPS cells were thawed into a T12.5 flask containing feeder cells and cultured for two days to reach confluency. The day before injection, the cells were passed 1:2 or 1:3 into new T12.5 flasks and cultured overnight to provide exponentially growing cells for blastocyst injection. The iPS cells were trypsinized to give a single cell suspension just prior to injection. Blastocysts were obtained at day 3.5 of gestation from superovulated albino C57B1/6J (B6(Cg)-*Tyr^{c-2J}*/J female mice which had been mated to stud males of the same strain (Jackson Laboratories) and injected with an average of 8 to 12 iPS^{OMO} cells to produce the chimeric embryos. After injection, blastocysts were returned to optimization medium and placed at 37 °C until transferred to recipient females. Ten to fifteen injected blastocysts were transferred to each uterine horn of 2.5-d-postcoitum-pseudopregnant females. This strain combination allowed identification of the contribution by the iPS cells to the chimeras based on coat color, since the iPS cells are derived from a C57B1/6J (black) mouse strain and the blastocysts obtained from an albino strain.

### B. Results

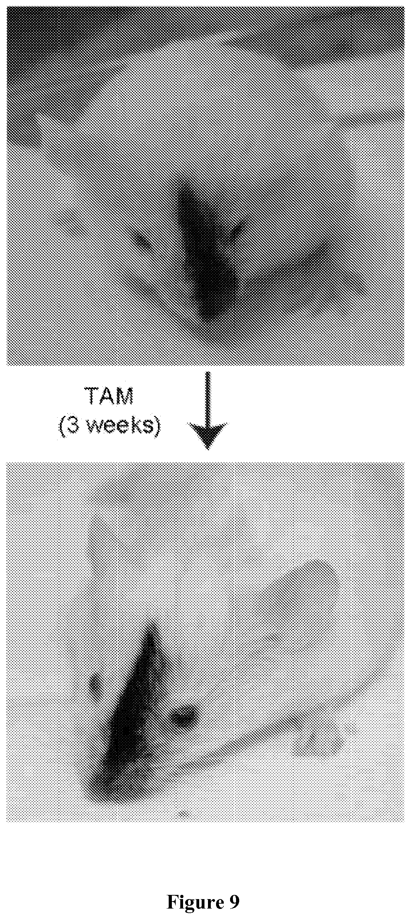

Since MYC has essential roles in normal proliferating cells and its temporary inactivation might have detrimental effects (Davis 1993), the long-term effect of MYC inactivation on healthy iPS derived tissues was examined. The chimeric mice produced by injecting two clones of karyotypically normal, iPS^{OMO} cells from C57B1/6J strain (black donor coat) into blastocysts of C57B1/6J (B6(Cg)-*Tyr^{c-2j}*/J strain (white recipient coat) readily allowed identification of chimeric animals (∼20%) and was a first indication of their level of chimerism (Fig. 9). MYC blockade was induced using the tamoxifen treatment protocol described in Example 3 (10mg/kg on alternate days for 3 weeks). The treated and untreated animals were equally active, maintained feeding and grooming habits and showed no weight loss. Further, no obvious effect of MYC blockade was observed on the black, iPS cell-derived skin and fur (Fig. 9). Regenerative tissues in Omomyc-transgenic animals showed anti-proliferative effects such as loss of the gastrointestinal villi, hair loss, and reduced cellularity in the hematopoietic system, and these changes were reversible within 4 weeks (Soucek 2008). In chimeric mice, tissues derived from iPS^{OMO} cells were readily recognizable by immunohistochemical detection of the co-expressed citrine reporter. The effects of MYC inactivation on iPS-derived and host tissues were compared 4 weeks after treatment, including hair follicles, dermal and epidermal structures, the upper gastrointestinal tract and the thyroid gland, the kidney, lower gastrointestinal tract and its mucosal villi, and red and white pulp of the spleen. There were extensive contributions of iPS^{OMO}-derived cells in these sites, but, importantly, no differences were observed in microscopic anatomy between iPS^{OMO} and host-derived organ structures following tamoxifen treatment. Hence, prolonged MYC inactivation did not produce lasting organ damage in iPS-derived tissues and was well tolerated in a physiological context.

### EXAMPLE 6

### Treatment of Leukemia with Omomyc^{ER}

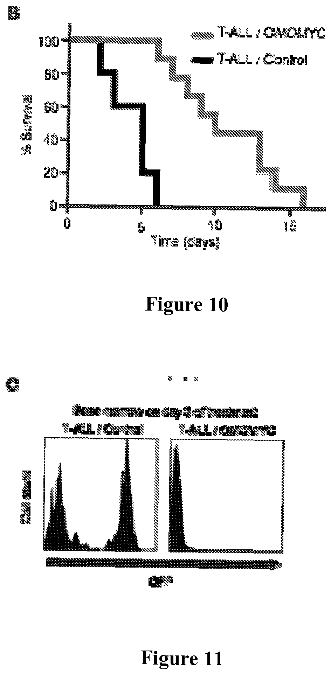

A cell line expressing intracellular Notch (ICN) was transduced with Omomyc^{ER} or control vector, and clones were grown in culture. Cells were harvested and injected into mice, leading to rapid development of T cell acute lymphoblastic leukemia (T-ALL) in mice. Fig. 10 shows the survival of the T-ALL mice in Omomyc mice and control mice treated with tamoxifen. Fig. 11 shows a FACS plot of cells from the bone marrow of T-ALL/Omomyc and T-ALL/control mice on day 3 of tamoxifen treatment. These results indicate that Omomyc can drive aggressive leukemias arising from transplanted hematopoietic cells (bone marrow or fetal liver) cells into remission. The effect of Omomyc on non-leukemic hematopoietic cells (such as those derived from transplanted bone marrow or fetal liver) can confirm that Omomyc is not deleterious for these cells and that reconstitution of the hematopoietic system is not impaired upon transplantation.

### EXAMPLE 7

### Generation and treatment of iPS-derived Neural Tumors

### A. Differentiation of iPS cells

### 1. Neural rosettes

Control iPSs and omomyc^{ER} iPSs were differentiated towards a neural fate by a modified dual SMAD-inhibition protocol (Smith 2008). After 8 to 10 days of differentiation, iPSs lines were subjected to WNT signaling inhibition (XAV939: 10 µM) to prevent non-CNS neural fates. Four days after passaging, neural cultures formed polarized columnar neuroepithelial structures known as neural rosettes. These cells were dissociated and collected, and a total of 200,000 cells were injected into the striatum of immunocompromised mouse host (see grafting section below).

### 2. Midbrain dopamine (DA) neurons

Midbrain dopaminergic neurons were derived as previously described (Lin 2009). Briefly, iPS cell lines were exposed to activators of SHH (puromorphamine) and WNT signaling (GSK-inhibitor: CHIR99021) to induce FOXA2+/LMX1A+ midbrain floor plate cells. These cells were further differentiated into to DA neurons in Neuralbasal/B27 medium supplemented with BDNF, ascorbic acid, GDNF, dibutyryl-cyclic AMP and TGFβ3. At day 23 of DA neuron differentiation, cells were infected with the Omomyc^{ER} virus for 48 h and FACS-purified for citrine-positive cells. A total of 150,000 cells were injected into the striatum of immunocompromised mouse host (see grafting section below). Prior to transplantation, DA neuron identity and induction efficiency was validated by immunocytochemistry for co-expression of FOAX2/LMX1A/NURR1.

### B. Grafting.

*NOD.Cg-Prkdc^{scld} Il2rg^{tm1 Wjl}*/SzJ (The Jackson Laboratory), 6 to 8 weeks old, were anesthetized with ketamine/xylazine (Fort Dodge), and unilateral stereotactic injections of hESC-derived cells were delivered to the striatum at the following coordinates: anteroposterior [AP], +0.5; mediolateral [ML], -1.8; dorsoventral [DV], -3.2. Transplantation of 150,000 (mDA) or 200,000 cells (rosettes), at a concentration of 100,000 cells/µl, was performed with a Hamilton syringe (26s gauge) at a rate of one µl per minute.

### C. Immunohistochemistry.

Mice were given a lethal dose of pentobarbital (Nembutal, Abbot Laboratories) and transcardially perfused with PBS followed by 4% paraformaldehyde. Brains were removed and fixed overnight at 4°C and then transferred to 30% sucrose overnight. Brains were embedded in O.C.T. (Tissue-Tek) and stored at -80°C until serial cryosectioning at 30 microns in 5 bottles. For fluorescence immunohistochemistry, sections were blocked in 10% normal goat serum (NGS; Gibco) (1% BSA for FOXA2 staining) with 0.2% Triton X-100 for 30 minutes at room temperature. Primary antibodies were applied overnight in 2% NGS (.1% BSA for Sox2 and FOXA2 staining) at 4°C, followed by appropriate fluorochrome-conjugated secondary antibodies (Alexa Fluor conjugates; Molecular Probes) for 1 hour at room temperature. Slides were washed and counterstained with Vectashield with DAPI (Vector Laboratories). Primary antibodies included mouse anti-nestin (1:500; Neuromics), goat anti-Sox2 (1:100; Santa Cruz), mouse anti-Ki67 (1:500; DAKO), rabbit anti-TH (1:500; Pel Freez Biologicals), goat anti-FOXA (1:100; Santa Cruz Biotechnology Inc.), and mouse anti-hNCAM (ERIC-1; 1:100; Santa Cruz).

### D. Results

Omomyc^{ER} was tested in DA neurons and primitive neural tumors arising from human iPS-derived neural rosette cells. Human iPS cells were generated using a single and excisable polycistronic vector expressing OCT4, KLF4, cMYC and SOX2 (Papapetrou 2011b). These cells were differentiated to neural precursors (rosette cell stage) and differentiated to midbrain dopaminergic (DA) neurons as described (Kriks 2011; Chambers 2009). These cells were engineered to express Omomyc^{ER} or a control vector. Intracerebral injection of rosette cells led to the development of brain tumors within 4 weeks (vector n=4 and Omomyc^{ER} n=5). Omomyc^{ER} activation by tamoxifen (TAM, 10 mg/ml twice a week for 5 weeks) completely blocked the growth of these brain tumors while animals bearing control tumors showed progressive tumor growth by magnetic resonance imaging (MRI). Pathologically, the tumors resembled primitive neuro-ectodermal tumors (PNET) and expressed Sox2 and Nestin but lacked more specific markers of neuronal or glial differentiation (Olig2, NeuN, synaptophysin or GFAP). MYC inactivation caused further loss of primitive markers (Sox2, Nestin) and did not induce tumor cell differentiation. Histology showed that the dense islands of proliferating (Ki67 positive) cells disappeared completely upon Omomyc^{ER} induction. Hence, MYC inhibition blocks the growth of PNET that can result from aberrant differentiation of human iPS cells *in vivo.*

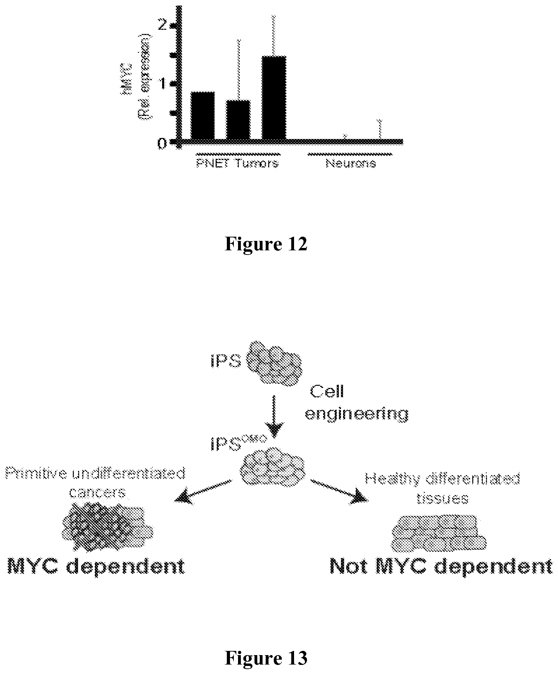

The effect of Omomyc^{ER} induction on iPS-derived midbrain DA neurons injected into the mouse brain was tested using the same tamoxifen treatment schedule as for the brain cancers. MRI showed equal engraftment of Omomyc^{ER} and control cells. Microscopically, both omomyc^{ER} and control neurons produced a well-defined graft core composed of TH+ cells co-expressing the transcription factor FOXA2 typical for midbrain DA neurons and human-specific NCAM. Consistent with the differential MYC requirement in PNETs and normal Dneurons, qRT-PCR revealed elevated MYC expression in tumors and undetectable MYC levels in differentiated neurons (Fig. 12). Hence, temporary *MYC* inactivation distinguishes primitive and undifferentiated cancers from healthy, differentiated human iPS-derived tissues (Fig. 13).

### REFERENCES

Chambers, S.M., et al. Highly efficient neural conversion of human ES and iPS cells by dual inhibition of SMAD signaling. Nat. Biotechnol 27, 275-280 (2009).
Davis, A. C., Wims, M., Spotts, G. D., Hann, S. R. & Bradley, A. A null c-myc mutation causes lethality before 10.5 days of gestation in homozygotes and reduced fertility in heterozygous female mice. Genes Dev 7, 671-682, (1993).
Felsher, D. W. & Bishop, J. M. Reversible tumorigenesis by MYC in hematopoietic lineages. Mol. Cell 4, 199-207, (1999).
Fukazawa et al. Inhibition of MYC Effectively Targets KRAS Mutation-positive Lung Cancer Expressing High Levels of MYC. Anticancer Res. 30:4193-4200 (2010).
Hanna, J. H., Saha, K. & Jaenisch, R. Pluripotency and Cellular Reprogramming: Facts, Hypotheses, Unresolved Issues. Cell 143, 508-525, (2010).
Hong, H. Suppression of induced pluripotent stem cell generation by the p53-p21 pathway. Nature 460, 1132-1135, (2009).
Jain, M. et al. Sustained Loss of a Neoplastic Phenotype by Brief Inactivation of MYC. Science 297, 102-104, (2002).
Kawamura, T. Linking the p53 tumour suppressor pathway to somatic cell reprogramming. Nature 460, 1140-1144, (2009).
Kriks, S, Shim, J.W., Piao J., Ganat, Y.M., Wakeman, D.R., Xie, Z., Carrillo-Reid, L., Auyeung, G., Antonacci, C., Buch, A., Yang, L., Beal, M.F., Surmeier, D.J., Kordower, J.H., Tabar, V. & Studer, L. Dopamine Neurons Derived from Human ES Cells Efficiently Engraft in Animal Models of Parkinson's Disease. Nature 480, 547-51 (2011).
Knoepfler, P. S. Deconstructing Stem Cell Tumorigenicity: A Roadmap to Safe Regenerative Medicine. Stem Cells 27, 1050-1056, (2009).
Lin, C.H., Jackson, A.L., Guo, J., Linsley, P.S. & Eisenman, R.N. Myc-regulated microRNAs attenuate embryonic stem cell differentiation. Embo J. 28, 3157-3170 (2009).
Littlewood, T.D., Hancock, D.C., Danielian, P.S., Parker, M. G., & Evan, G.I. A Modified Oestrogen Receptor Ligand-binding Domain as an Improved Switch for the Regulation of Hereologous Proteins. Nucleic Acids Res. 23, 1686-1690 (1995).
Marion, R. M. et al. A p53-mediated DNA damage response limits reprogramming to ensure iPS cell genomic integrity. Nature 460, 1149-1153, (2009).
Meyer, N. & Penn, L.Z. Reflecing on 25 years with MYC. Nature Rev. Cancer 8, 976-990 (2008).
Nakagawa, M., Takizawa, N., Narita, M., Ichisaka, T. & Yamanaka, S. Promotion of direct reprogramming by transformation-deficient MYC. Proc Natl Acad Sci USA 107, 14152-14157, (2010).
Okita, K., Ichisaka, T. & Yamanaka, S. Generation of germline-competent induced pluripotent stem cells. Nature 448, 313-317, (2007).
Papapetrou, E. P. et al. Stoichiometric and temporal requirements of Oct4, Sox2, Klf4, and c-MYC expression for efficient human iPSC induction and differentiation. Proc Natl Acad Sci USA 106, 12759-12764, (2009).
Papapetrou, E. P. et al. Genomic safe harbors permit high beta-globin transgene expression in thalassemia induced pluripotent stem cells. Nat Biotechnol 29, 73-78, (2011a).
Papapetrou, E.P. & Sadelain, M. Generation of trans gene-free human induced pluripotent stem cells with an excisable single polycistronic vector. Nat Protoc 6, 1251-1273 (2011b).
Savino et al. the Action Mechanism of the MYC Inhibitor Termed Omomyc May Give Clues on How to Target MYC for Cancer Therapy. PLoS One 6, e22284 (Epub 2011)
Schuldiner, M., Itskovitz-Eldor, J. & Benvenisty, N. Selective ablation of human embryonic stem cells expressing a "suicide" gene. Stem cells 21, 257-265, (2003).
Shachaf, C. M. et al. MYC inactivation uncovers pluripotent differentiation and tumour dormancy in hepatocellular cancer. Nature 431, 1112-1117, (2004).
Shih, C. C., Forman, S. J., Chu, P. & Slovak, M. Human embryonic stem cells are prone to generate primitive, undifferentiated tumors in engrafted human fetal tissues in severe combined immunodeficient mice. Stem Cells Dev 16, 893-902, (2007).
Smith, K.N., Singh, A.M. & Dalton, S. Myc Represses Primitive Endoderm Differentiation in Pluripotent Stem Cells. Cell Stem Cell 7, 343-354 (2010).
Sodir N. et al. Endogenous MYC Maintains the Tumor Microenvironment. Genes Dev. 25:907-916 (2011).
Soucek, L. Design and properties of a MYC derivative that efficiently homodimerizes. Oncogene 17, 2463-2472, (1998).
Soucek, L. et al. Modelling MYC inhibition as a cancer therapy. Nature 455, 679-683, (2008).
Soucek, L. Omomyc, a potential MYC dominant negative, enhances MYC-induced apoptosis. Cancer Res. 62, 3507-3510, (2002).
Soucek, L., Nasi, S. & Evan, G. I. Omomyc expression in skin prevents MYC-induced papillomatosis. Cell Death Differ. 11, 1038-1045, (2004).
Takahashi, K. & Yamanaka, S. Induction of pluripotent stem cells from mouse embryonic and adult fibroblast cultures by defined factors. Cell 126, 663-676, (2006).
Van Eyss, B. & Eilers, M. Addicted to MYC--But Why? Genes Dev. 25895-897 (2011).
Wernig, M. et al. In vitro reprogramming of fibroblasts into a pluripotent ES-cell-like state. Nature 448, 318-324, (2007).
Wernig, M., Meissner, A., Cassady, J. P. & Jaenisch, R. c-MYC is dispensable for direct reprogramming of mouse fibroblasts. Cell Stem Cell 2, 10-12, (2008).

## Claims

1. An agent for use in reducing tumorigenesis or tumor growth in a MYC-dependent cancer occurring during the course of gene therapy in a subject,
wherein the subject has been administered cells that have been transduced ex *vivo* with a gene therapy vector comprising a therapeutic gene operably linked to a first control element, and a dominant-negative MYC-interfering protein (D-MIP) gene operably linked to an inducible control element,
wherein the MYC-dependent cancer is derived from the cells that have been administered to the subject for gene therapy,
wherein the agent is an inducer of the inducible control element, and
wherein, upon detection of cancer in the subject, the inducer is administered to the subject for a time and in an amount to activate D-MIP in the subject, thereby reducing tumorigenesis or tumor growth of the MYC-dependent cancer.

2. The agent for use according to claim 1 wherein the inducible control element is a mutant hormone binding domain of the estrogen receptor and the inducer is tamoxifen.

3. The agent for use according to claim 1 wherein the inducible control element is a cumate switch inducible system and the inducer is cumate; the inducible control element is a glucocorticoid-responsive promoter and the inducer is a glucocorticoid; the inducible control element is a tetracycline response element and the inducer is a tetracycline such as doxycycline; the inducible control element is a hormone receptor ligand binding domain and the inducer is a hormone receptor ligand; or wherein the inducible control element is an HSV viral protein VP16 activator domain and the inducer is an HSV VP16 activator protein.

4. The agent for use according to any of claims 1-3 wherein the gene therapy vector is a lentiviral vector for treating severe combined immunodeficiency disease (SCID) or for treating a hemoglobinopathy such as thalassemia or sickle cell disease.

5. The agent for use according to any of claims 1-3 wherein the MYC-dependent cancer is bladder cancer, breast cancer, colon cancer, gastric cancer, hepatocellular carcinoma, leukemia, lymphoma, melanoma, myeloma such as multiple myeloma, neuroblastoma, ovarian cancer, prostate cancer, rhabdomyosarcoma, small cell lung cancer, subungual melanoma, or uveal melanoma, and wherein the Myc-dependent cancers is metastatic or non-metastatic.

6. The agent for use according to any of claims 1-5, wherein said gene therapy vector is a lentiviral vector, an adenoviral vector or an adeno-associated viral vector.

7. The agent for use according to any of claims 1-6, wherein said D-MIP is Omomyc or wherein said D-MIP and second control element together form Omomyc^{ER}.

8. The agent for use according to any of claims 1-7, wherein said cells are hematopoietic stem cells, hematopoietic progenitor cells, iPS cells or ES cells.

9. The agent for use according to claims 1-8, wherein said cells are autologous cells from said subject.

10. The agent for use according to any of claims 1-9, wherein said subject is human.

## Patentansprüche

1. Mittel zur Verwendung beim Verringern einer Tumorentstehung oder eines Tumorwachstums bei einer MYC-abhängigen Krebserkrankung, der im Verlauf einer Gentherapie bei einem Subjekt auftritt,
wobei dem Subjekt Zellen verabreicht wurden, die *ex vivo* mit einem Gentherapievektor, der ein therapeutisches Gen umfasst, das in Wirkbeziehung mit einem ersten Kontrollelement verknüpft ist, und einem Gen des dominant-negativen MYCinterferierenden Proteins (D-MIP), das in Wirkbeziehung mit einem induzierbaren Kontrollelement knüpft ist, transduziert wurden,
wobei die MYC-abhängige Krebserkrankung von den Zellen stammt, die dem Subjekt zur Gentherapie verabreicht wurden,
wobei das Mittel ein Induktor des induzierbaren Kontrollelements ist, und
wobei nach einer Erkennung einer Krebserkrankung bei dem Subjekt der Induktor an das Subjekt für eine Zeit und in einer Menge verabreicht wird, um D-MIP bei dem Subjekt zu aktivieren, wodurch eine Tumorentstehung oder ein Tumorwachstum der MYC-abhängigen Krebserkrankung verringert wird.

2. Mittel zur Verwendung nach Anspruch 1, wobei das induzierbare Kontrollelement eine mutierte Hormonbindungsdomäne des Östrogenrezeptors ist und der Induktor Tamoxifen ist.

3. Mittel zur Verwendung nach Anspruch 1, wobei das induzierbare Steuerelement ein mit einem Cuminatschalter induzierbares System ist und der Induktor Cuminat ist; das induzierbare Kontrollelement ein auf Glucocorticoid ansprechender Promotor ist und der Induktor ein Glucocorticoid ist; das induzierbare Kontrollelement ein Tetracyclin-Ansprechelement ist und der Induktor ein Tetracyclin wie Doxycyclin ist; das induzierbare Kontrollelement eine Hormonrezeptorligandenbindungsdomäne ist und der Induktor ein Hormonrezeptorligand ist; oder wobei das induzierbare Kontrollelement eine HSV-Virusprotein-VP16-Aktivatordomäne ist und der Induktor ein HSV-VP16-Aktivatorprotein ist.

4. Mittel zur Verwendung nach einem der Ansprüche 1-3, wobei der Gentherapievektor ein lentiviraler Vektor zum Behandeln einer schweren kombinierten Immunschwächekrankheit (SCID) oder zum Behandeln einer Hämoglobinopathie wie Thalassämie oder Sichelzellenkrankheit ist.

5. Mittel zur Verwendung nach einem der Ansprüche 1-3, wobei die MYC-abhängige Krebserkrankung Blasenkrebs, Brustkrebs, Dickdarmkrebs, Magenkrebs, hepatozelluläres Karzinom, Leukämie, Lymphom, Melanom, Myelom wie multiples Myelom, Neuroblastom, Eierstockkrebs, Prostatakrebs, Rhabdomyosarkom, kleinzelliger Lungenkrebs, subunguales Melanom oder uveales Melanom ist, und wobei die Myc-abhängigen Krebserkrankungen metastatisch oder nicht metastatisch sind.

6. Mittel zur Verwendung nach einem der Ansprüche 1-5, wobei der Gentherapievektor ein lentiviraler Vektor, ein adenoviraler Vektor oder ein Adeno-assoziierter Virusvektor ist.

7. Mittel zur Verwendung nach einem der Ansprüche 1-6, wobei das D-MIP Omomyc ist oder wobei das D-MIP und das zweite Kontrollelement zusammen Omomyc^{ER} bilden.

8. Mittel zur Verwendung nach einem der Ansprüche 1-7, wobei die Zellen hämatopoetische Stammzellen, hämatopoetische Vorläuferzellen, iPS-Zellen oder ES-Zellen sind.

9. Mittel zur Verwendung nach den Ansprüchen 1-8, wobei die Zellen autologe Zellen von dem Subjekt sind.

10. Mittel zur Verwendung nach einem der Ansprüche 1-9, wobei das Subjekt ein Mensch ist.

## Revendications

1. Agent destiné à être utilisé dans la réduction de la tumorigenèse ou de la croissance tumorale dans un cancer dépendant de Myc survenant au cours d'une thérapie génique chez un sujet,
dans laquelle on a administré au sujet des cellules qui ont été transduites *ex vivo* avec un vecteur de thérapie génique comprenant un gène thérapeutique lié de manière opérationnelle à un premier élément de contrôle, et un gène exprimant une protéine interférant avec Myc (D-MIP) de type dominante négative lié de manière fonctionnelle à un élément de contrôle inductible,
dans lequel le cancer dépendant de Myc est dérivé de cellules qui ont été administrées au sujet pour une thérapie génique,
dans lequel l'agent est un inducteur de l'élément de contrôle inductible, et
dans lequel, lors de la détection d'un cancer chez le sujet, l'inducteur est administré au sujet pendant un temps et en quantité permettant d'activer la D-MIP chez le sujet, réduisant ainsi la tumorigenèse ou la croissance tumorale du cancer dépendant de Myc.

2. Agent à utiliser selon la revendication 1, dans lequel l'élément de contrôle inductible est un domaine mutant de liaison aux hormones du récepteur d'oestrogènes et l'inducteur est le tamoxifène.

3. Agent à utiliser selon la revendication 1, dans lequel l'élément de contrôle inductible est un système d'expression inductible au cumate et l'inducteur est le cumate ; l'élément de contrôle inductible est un promoteur sensible aux glucocorticoïdes et l'inducteur est un glucocorticoïde ; l'élément de contrôle inductible est un élément répondant à la tétracycline et l'inducteur est une tétracycline telle que la doxycycline ; l'élément de contrôle inductible est un domaine de liaison de ligand de récepteur d'hormone et l'inducteur est un ligand de récepteur d'hormone ; ou dans lequel l'élément de contrôle inductible est un domaine activateur VP16 de la protéine virale du HSV et l'inducteur est une protéine activatrice de la VP16 du HSV.

4. Agent à utiliser selon l'une quelconque des revendications 1 à 3, dans lequel le vecteur de thérapie génique est un vecteur lentiviral pour traiter un déficit immunitaire combiné sévère (SCID) ou pour traiter une hémoglobinopathie telle que la thalassémie ou la drépanocytose.

5. Agent à utiliser selon l'une quelconque des revendications 1 à 3, dans lesquelles le cancer dépendant de Myc est un cancer de la vessie, un cancer du sein, un cancer du côlon, un cancer de l'estomac, un carcinome hépatocellulaire, une leucémie, un lymphome, un mélanome, un myélome tel qu'un myélome multiple, un neuroblastome, un cancer de l'ovaire, un cancer de la prostate, un rhabdomyosarcome, un cancer du poumon à petites cellules, un mélanome subungual ou un mélanome uvéal, et dans lequel les cancers dépendants de Myc sont métastatiques ou non métastatiques.

6. Agent à utiliser selon l'une quelconque des revendications 1 à 5, dans lesquelles ledit vecteur de thérapie génique est un vecteur lentiviral, un vecteur adénoviral ou un vecteur viral adéno-associé.

7. Agent à utiliser selon l'une quelconque des revendications 1 à 6, dans lequel ledit D-MIP est Omomyc ou dans lequel ledit D-MIP et ledit deuxième élément de contrôle forment ensemble Omomyc^{ER}.

8. Agent à utiliser selon l'une quelconque des revendications 1 à 7, dans lequel lesdites cellules sont des cellules souches hématopoïétiques, des cellules progénitrices hématopoïétiques, des cellules iPS ou des cellules ES.

9. Agent à utiliser selon les revendications 1 à 8, dans lequel lesdites cellules sont des cellules autologues dudit sujet.

10. Agent à utiliser selon l'une quelconque des revendications 1 à 9, dans lequel ledit sujet est un être humain.
